# EUROPEAN PATENT SPECIFICATION

(11) **EP 2 908 778 B1**
(45) Date of publication and mention of the grant of the patent: **23.11.2016**
(21) Application number: 13780031.4
(22) Date of filing: 09.10.2013
(51) Int. Cl.: A61F 2/24, A61F 2/00, A61B 17/00

(54) **ANTI-THROMBUS FEATURE FOR IMPLANTED MEDICAL DEVICES**
ANTI-THROMBUS-FUNKTION FÜR IMPLANTIERTE MEDIZINISCHE VORRICHTUNGEN
DISPOSITIF ANTI-THROMBUS POUR DISPOSITIFS MÉDICAUX IMPLANTÉS

(30) Priority: 19.10.2012 US 201261716265 P
(43) Date of publication of application: 26.08.2015
(73) Proprietor: Boston Scientific Scimed, Inc., Maple Grove, MN 55311 (US)
(72) Inventor: PU, Zhou, Maple Grove, Minnesota 55311 (US); DONGMING, Hou, Plymouth, Minnesota 55446 (US); HUIBREGTSE, Barbara A., Westborough, Massachusetts 01581 (US); WEBER, Jan, 6228 GJ Maastricht (NL); AIDEN, Flanagan, Kilcolgan Co. Galway (IE)
(74) Representative: Vossius & Partner Patentanwälte Rechtsanwälte mbB
(86) International application number: PCT/US2013/064095
(87) International publication number: WO 2014/062448

(56) References cited:
- DE-A1-102010 044 746

## Description

### Cross-Reference to Related Applications

This application claims priority to U.S. Provisional Application No. 61/716,265 filed October 19, 2012.

### Technical Field

The disclosure relates generally to percutaneous medical devices and more particularly to percutaneous medical devices for implantation into the circulatory system.

### Background

Many permanently-implanted medical devices within the circulatory system, such as, for example, devices for implantation in a left atrial appendage, aortic valve replacement devices, or other implanted devices, may have exposed connections or connectors after delivery. In some patients, there is potential for thrombus to develop in, on, or around these exposed connections or connectors, which may disrupt normal blood flow and/or create areas of stagnant blood. Thrombi may break off into the bloodstream. Thrombi in the bloodstream may present a risk for stroke if the thrombi travel up the carotid arteries. Thrombi in the bloodstream may present a risk of blockage of smaller vessels, thereby depriving tissue/organs of proper oxygenation, if the thrombi travel farther downstream. A continuing need exists for improved medical devices and methods to control thrombus formation in, on, over, and around implanted medical devices.

### Summary

A medical device for permanent implantation in a circulatory system of a patient may include an implant configured for percutaneous or minimally-invasive insertion into the patient, at least one connector fixedly attached to the implant and configured to releasably attach to a delivery device, the at least one connector being exposed to blood flow within the circulatory system following implantation, and an anti-thrombus feature configured to transition from an undeployed state to a deployed state.

### Brief Description of the Drawings

Figure 1 is a schematic partial cross-sectional view of a heart;
Figure 2 is a schematic perspective view of an example implantable medical device;
Figure 2A is an enlarged schematic perspective view of an example connector of the example medical device of Figure 2;
Figure 3 is a schematic partial cross-sectional view of an example implantable medical device;
Figure 3A is an enlarged schematic partial cross-sectional view of an example connector of the example medical device of Figure 3;
Figure 4 is a schematic side view of an example implantable medical device;
Figure 4A is an enlarged schematic side view of an example connector of the example medical device of Figure 4;
Figure 5A illustrates an example connector including an example anti-thrombus feature in an undeployed state;
Figure 5B illustrates the example connector including the example anti-thrombus feature of Figure 5A in a deployed state;
Figure 6A illustrates an example connector in an installation condition including an example anti-thrombus feature in an undeployed state;
Figure 6B illustrates the example connector of Figure 6A in a delivered condition including the example anti-thrombus feature in an undeployed state;
Figure 6C illustrates the example connector of Figures 6A and 6B in a delivered condition including the example anti-thrombus feature in a deployed state;
Figure 7A illustrates an example connector in an installation condition including an example anti-thrombus feature in an undeployed state;
Figure 7B illustrates the example connector of Figure 7A in a delivered condition including the example anti-thrombus feature in a deployed state;
Figure 8A illustrates an example connector in an installation condition including an example anti-thrombus feature in an undeployed state;
Figure 8B illustrates the example connector of Figure 8A in a delivered condition including the example anti-thrombus feature in a deployed state;
Figure 9A illustrates an example connector in a partially delivered condition including an example anti-thrombus feature in an undeployed state;
Figure 9B illustrates the example connector of Figure 9A in a delivered condition including the example anti-thrombus feature in a deployed state;
Figure 10A illustrates an example connector in an installation condition including an example anti-thrombus feature in an undeployed state; and
Figure 10B illustrates the example connector of Figure 10A in a partially delivered condition including the example anti-thrombus feature in a partially deployed state.

While the invention is amenable to various modifications and alternative forms, specifics thereof have been shown by way of example in the drawings and will be described in greater detail below. It should be understood, however, that the intention is not to limit the invention to the particular embodiments described. On the contrary, the intention is to cover all modifications, equivalents, and alternatives falling within the spirit and scope of the invention.

### Detailed Description

For the following defined terms, these definitions shall be applied, unless a different definition is given in the claims or elsewhere in this specification.

The terms "upstream" and "downstream" refer to a position or location relative to the direction of blood flow through a particular element or location, such as a vessel (i.e., the aorta) or vessel lumen, a heart valve (i.e., the aortic valve), and the like.

All numeric values are herein assumed to be modified by the term "about," whether or not explicitly indicated. The term "about", in the context of numeric values, generally refers to a range of numbers that one of skill in the art would consider equivalent to the recited value (i.e., having the same function or result). In many instances, the term "about" may include numbers that are rounded to the nearest significant figure. Other uses of the term "about" (i.e., in a context other than numeric values) may be assumed to have their ordinary and customary definition(s), as understood from and consistent with the context of the specification, unless otherwise specified.

Weight percent, percent by weight, wt%, wt%, % by weight, and the like are synonyms that refer to the concentration of a substance as the weight of that substance divided by the weight of the composition and multiplied by 100.

The recitation of numerical ranges by endpoints includes all numbers within that range, including the endpoints (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The various individual elements described below, even if not explicitly shown in a particular combination, are nevertheless contemplated as being combinable or arrangable with each other to form other additional embodiments or to complement and/or enrich the described embodiment(s), as would be understood by one of ordinary skill in the art.

Diseases and/or medical conditions that impact the cardiovascular system are prevalent in the United States and throughout the world. Traditionally, treatment of the cardiovascular system was often conducted by directly accessing the impacted part of the system. For example, treatment of a blockage in one or more of the coronary arteries was traditionally treated using coronary artery bypass surgery. As can be readily appreciated, such therapies are rather invasive to the patient and require significant recovery times and/or treatments. More recently, less invasive therapies have been developed, for example, where a blocked coronary artery could be accessed and treated via a percutaneous catheter (e.g., angioplasty). Such therapies have gained wide acceptance among patients and clinicians.

Some relatively common medical conditions may include or be the result of inefficiency, ineffectiveness, or complete failure of one or more of the valves within the heart. For example, failure of the aortic valve can have a serious effect on a human and could lead to a serious health condition and/or death if not dealt with. A human heart includes several different heart valves, including aortic, pulmonary, mitral, and tricuspid valves, which control the flow of blood to and from the heart. Over time, a heart valve may become obstructed, narrowed, and/or less flexible (i.e., stenosed) due to hardening, calcium deposition, or other factors, thereby reducing the flow of blood through the valve and/or increasing the pressure within the chambers of the heart as the heart attempts to pump the blood through the vasculature. One traditional treatment method is valve replacement, where the stenosed valve is removed and a replacement tissue or mechanical valve is implanted via open-heart surgery. Alternative treatments, including percutaneous valve replacement procedures (i.e., transcatheter aortic valve implantation, or TAVI) which may deliver and implant a replacement heart valve (i.e., aortic valve), have been developed which may be much less invasive to the patient.

The occurrence of thrombi in the left atrial appendage (LAA) during atrial fibrillation may be due to stagnancy of the blood pool in the LAA. In an effort to reduce the occurrence of thrombi formation within the left atrial appendage during atrial fibrillation, certain medical devices have been developed that close off the left atrial appendage from the heart and/or circulatory system. By reducing or elimination the stagnant pooling of blood, the formation of thrombi can be significantly reduced or avoided, thereby lowering the risk of stroke due to thrombolytic material entering the blood stream from the left atrial appendage.

Each of the medical devices used above, as well as others, may include connectors that have irregular surfaces and/or open apertures that disrupt the normal flow of blood and/or create areas of stagnant blood. The devices and methods described herein may provide additional desirable features and benefits for use with such devices.

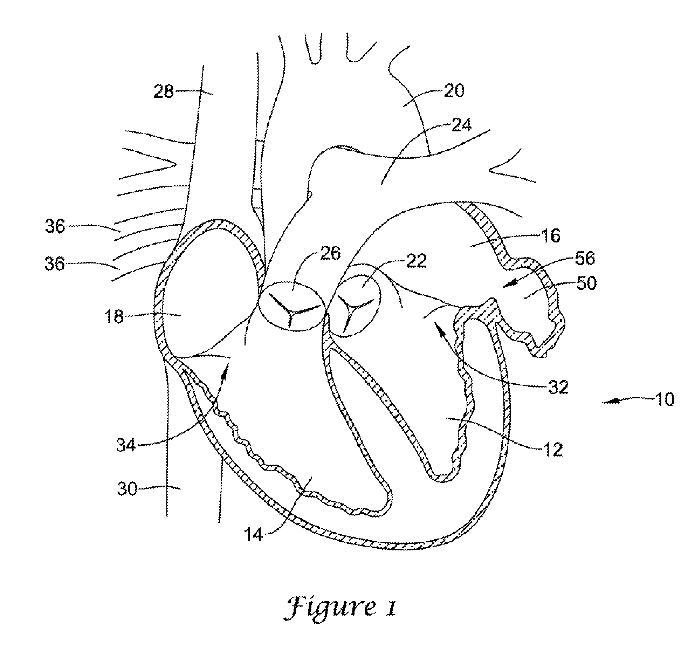

Turning to the drawings, Figure 1 is a partial cross-sectional view of certain elements of a typical human heart 10 and some immediately adjacent blood vessels. A heart 10 may include a left ventricle 12, a right ventricle 14, a left atrium 16, and a right atrium 18. An aortic valve 22 is disposed between the left ventricle 12 and an aorta 20. A pulmonary or semi-lunar valve 26 is disposed between the right ventricle 14 and a pulmonary artery 24. A superior vena cava 28 and an inferior vena cava 30 return blood from the body to the right atrium 18. A mitral valve 32 is disposed between the left atrium 16 and the left ventricle 12. A tricuspid valve 34 is disposed between the right atrium 18 and the right ventricle 14. Pulmonary veins 36 return blood from the lungs to the left atrium 16. A left atrial appendage (LAA) 50 is attached to and in fluid communication with the left atrium 16 via an ostium 56.

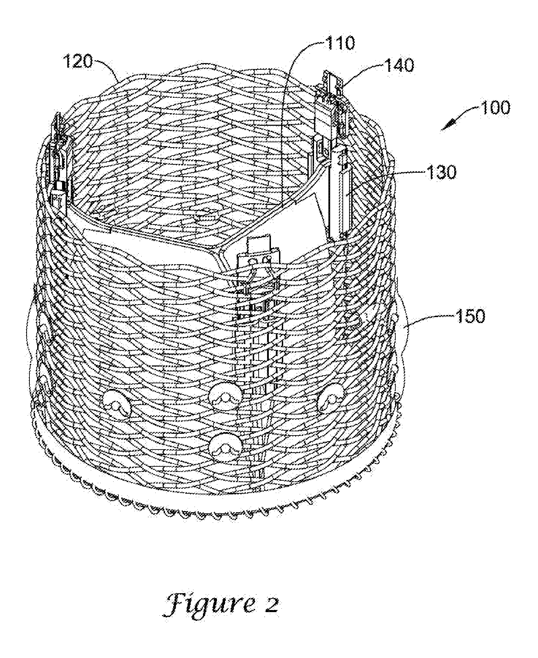

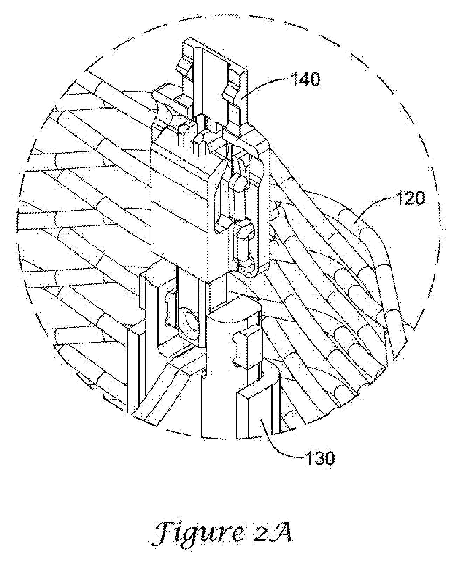

Figure 2 illustrates an example medical device such as an aortic valve replacement device 100 used in transcatheter aortic valve implantation (TAVI). In some embodiments, an aortic valve replacement device 100 may include a plurality of valve leaflets 110 each secured to a cylindrical braid 120 at a post 130. In some embodiments, each post 130 may be secured to the cylindrical braid 120, along an inside surface of the cylindrical braid 120 for example, with sutures, adhesives, or other suitable means. In some embodiments, each post 130 may include a connector 140 positioned adjacent to, longitudinally spaced from, and/or aligned with its respective post 130. Each connector 140 may be secured to the cylindrical braid 120, for example, by sutures, adhesives, or other suitable means. In some embodiments, the aortic valve replacement device 100 may also include a seal 150 disposed about the cylindrical braid 120. In some embodiments, the structure and/or operation of the connector 140 may be similar to a device disclosed in U.S. Application No. 61/559,941, filed November 15, 2011, and/or U.S. Application No. 61/577,880, filed December 20, 2011, both of which are herein incorporated by reference in their entirety.

In some embodiments, each connector 140 may be configured to releasably attach to a delivery device (not shown) in an installation condition. The installation condition generally includes the aortic valve replacement device 100 being disposed in a collapsed and/or elongated state. In the installation condition, the aortic valve replacement device 100 may be inserted (percutaneously, in some embodiments) into the aortic valve 22, where the aortic valve replacement device 100 may be expanded or actuated into the delivered condition. Figure 2 illustrates the aortic valve replacement device 100 in a delivered condition. Each connector 140 may include an irregular shape, as seen in Figure 2A for example, configured to matingly align with and/or attach to the delivery device. Upon actuation into the delivered condition, the delivery device may be removed, leaving each connector 140 with the irregular shape exposed to blood flow through the aortic valve 22 (and/or the aortic valve replacement device 100) and/or the aorta 20. With some medical devices, an irregular shape may disrupt blood flow and/or create an area of stagnant blood which may facilitate thrombus formation.

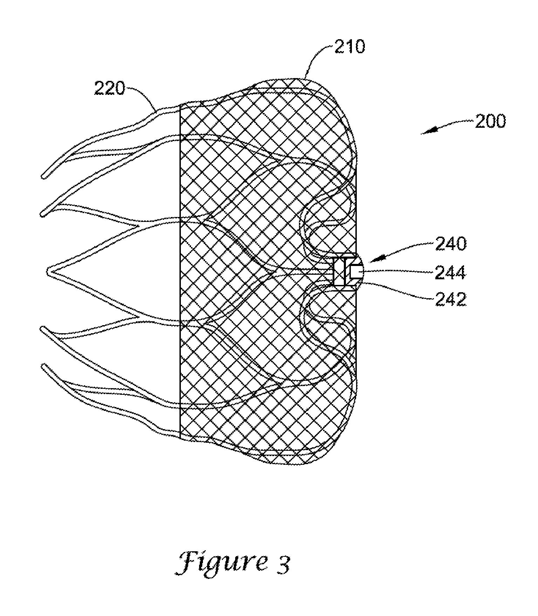

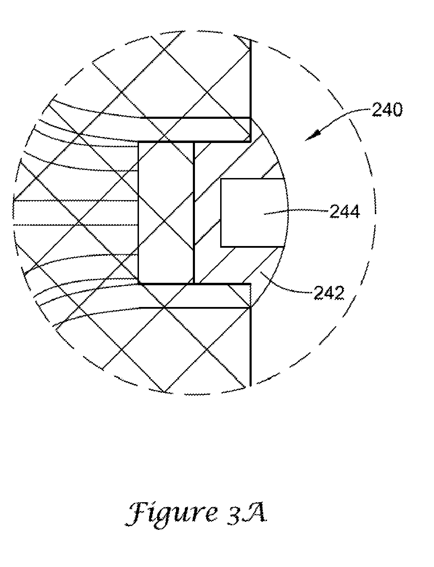

Figure 3 illustrates an example medical device such as an expandable filter device 200 used to close off a left atrial appendage 50 from blood flow through the left atrium 16. In some embodiments, an expandable filter device 200 may include a filter membrane 210 supported on an elastic wire frame 220. In some embodiments, the filter membrane 210 may be impermeable to blood or the filter membrane 210 may be blood permeable while preventing thrombi or embolic debris from passing through the filter membrane 210. In some embodiments, the expandable filter device 200 may include a connector 240 having an insert 242 with a socket 244 disposed therein. In some embodiments, the socket 244 may be formed integrally with or directly into the elastic wire frame 220, with no insert 242 present or needed. In some embodiments, the structure and/or operation of the expandable filter device 200 may be similar to a device disclosed in U.S. Application No. 10/351,736, filed January 24, 2003, which is herein incorporated by reference in its entirety.

In some embodiments, the connector 240 may be configured to releasably attach to a delivery device (not shown) in an installation condition. The installation condition generally includes the expandable filter device 200 being disposed in a collapsed and/or elongated state. In the installation condition, the expandable filter device 200 may be inserted (percutaneously, in some embodiments) into an ostium 56 of a left atrial appendage 50, where the expandable filter device 200 may be expanded or actuated into the delivered condition. Figure 3 illustrates the expandable filter device 200 in a delivered condition. In some embodiments, the connector 240 may be configured to matingly align with and/or attach to the delivery device. Upon actuation into the delivered condition, the delivery device may be removed, leaving the socket 244 of the connector 240 empty, as seen in Figure 3A for example, and exposed to blood flow through the left atrium 16. In some embodiments, the connector 240 and/or the socket 244 may include threads, spring clips, notches, and/or other releasable interlocking features disposed therein. With some medical devices, an open aperture or socket 244 may disrupt blood flow and/or create an area of stagnant blood which may facilitate thrombus formation.

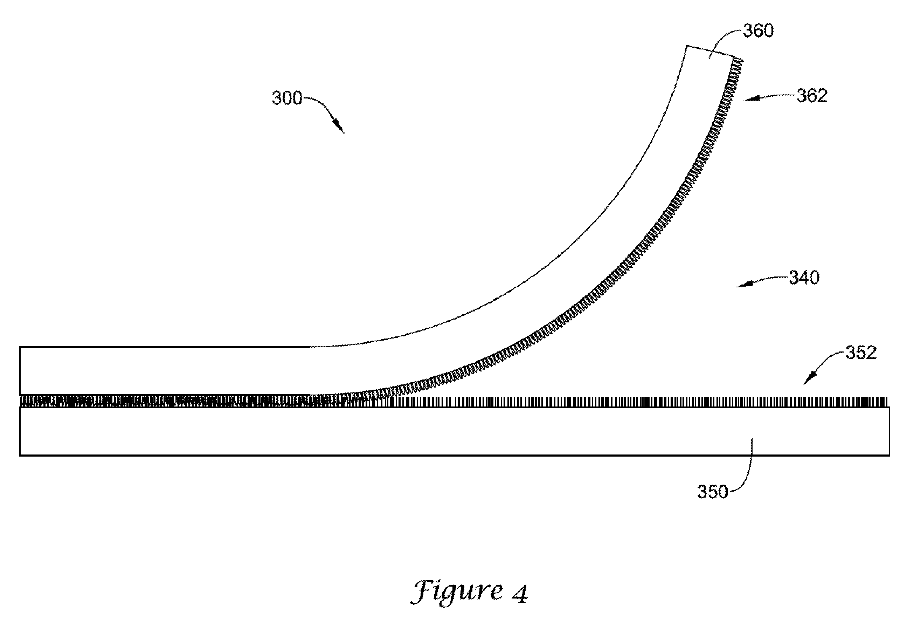

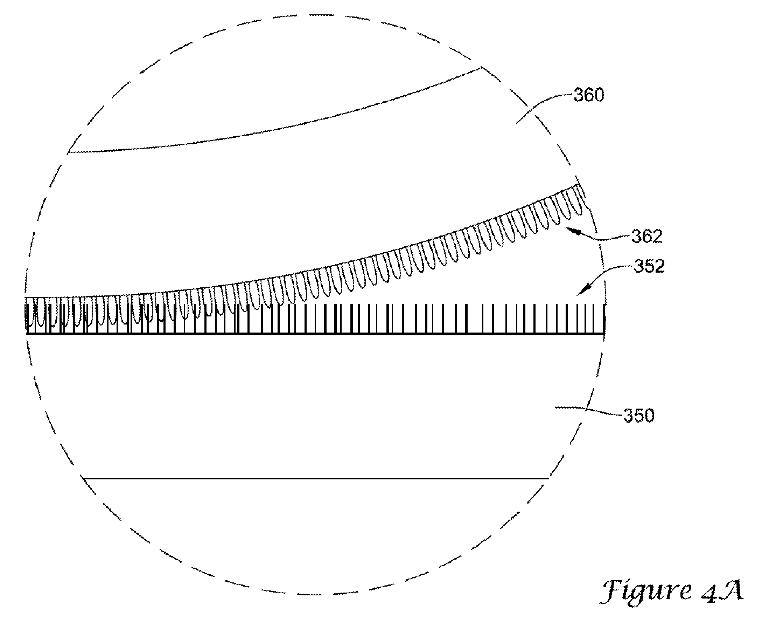

Figure 4 illustrates a portion of an example medical device 300 including an example connector 340 consisting of mating pieces of hook-and-loop material, such as Velcro®. As shown in Figure 4A, the connector 340 may comprise a first piece of material 350 including a plurality of hooks 352 and a second piece of material 360 including a plurality of loops 362. In some embodiments, the medical device 300 may be inserted (percutaneously, in some embodiments) into a patient in an installation condition, wherein the plurality of hooks 352 of the first piece of material 350 is matingly engaged with the plurality of loops 362 of the second piece of material 360. Following insertion, the medical device may be actuated into a delivered condition, wherein the first piece of material 350 is separated from the second piece of material 360. In the delivered condition, the second piece of material 360 may be removed from the patient, leaving the plurality of hooks 352 of the first piece of material 350 exposed to blood flow as an irregular shape, which may disrupt blood flow and/or create an area of stagnant blood which may facilitate thrombus formation. As may be appreciated, in some embodiments, the first piece of material 350 and the second piece of material 360 may be reversed, such that the first piece of material 350 may be removed from the patient, leaving the plurality of loops 362 of the second piece of material 360 exposed to blood flow as an irregular shape, which may disrupt blood flow and/or create an area of stagnant blood which may facilitate thrombus formation.

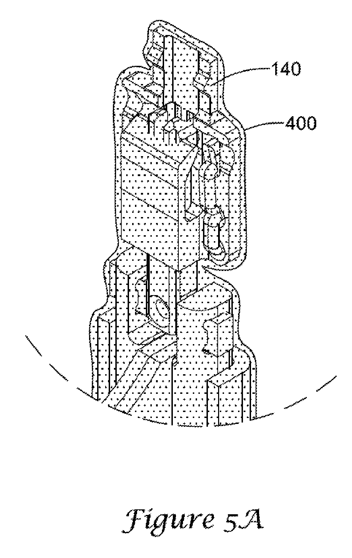

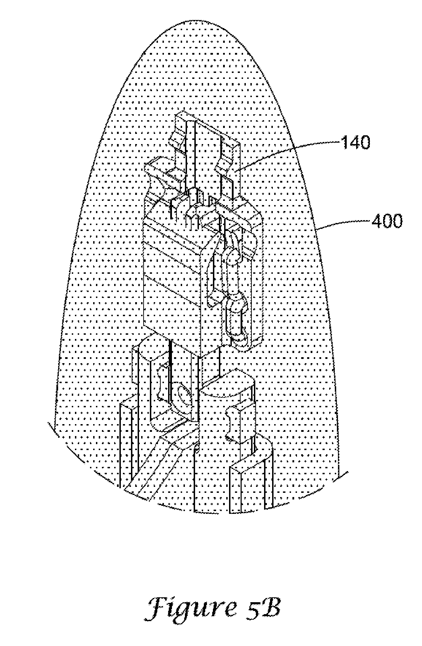

Figure 5A illustrates the connector 140 of Figures 2 and 2A above including an anti-thrombus feature 400. As illustrated, the anti-thrombus feature 400 is shown as a thin coating disposed in, on, over, and/or around the irregular shape of the connector 140 in an undeployed state. In some embodiments, the anti-thrombus feature 400 may be disposed on surfaces of the connector 140 that are only exposed, for example to surrounding fluid(s), after the delivery device (not shown) has been detached and/or removed. In some embodiments, the anti-thrombus feature 400 may include a swellable hydrogel and/or a water-swellable polymer that swells when it becomes hydrated (i.e., when the polymer is exposed to an aqueous environment such as body fluid(s), blood, and/or tissue). Some examples of swellable polymers may include polyvinyl alcohol, polyvinylpyrrolidone (PVP), polyethylene glycol, polyethylene oxide, hydroxypropyl methylcellulose, poly(hydroxyalkyl methacrylate) polyacrylic acid, coatings made of or including charged polymers, and the like. In some embodiments, the anti-thrombus feature 400 may swell and/or increase in volume upon exposure to fluid(s), such as blood or water, to a deployed state, as may be seen in Figure 5B. In some embodiments, the anti-thrombus feature 400 in the deployed state may increase in volume by 50%, 75%, 100%, 200%, 400%, 1000%, or more, upon exposure to fluid(s) compared to the undeployed state. In use, following insertion of aortic valve replacement device 100 and actuation into the delivered condition, the delivery device (not shown) may be detached from the connector(s) 140 as seen in Figure 5A, thereby exposing the anti-thrombus feature 400 to blood flow. The anti-thrombus feature 400 may then swell and/or increase in volume, in response to exposure to fluid (i.e., blood), to the deployed state, as may be seen in Figure 5B, to provide a smooth, protective surface over the irregular shape which may reduce the potential for thrombus formation.

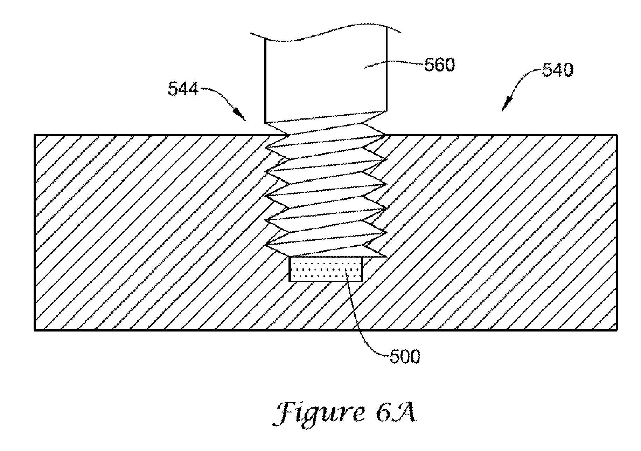

One of skill in the art will appreciate that the anti-thrombus feature 400 described herein is not limited to use on the connector 140, but may be equally applicable to any connector having an irregular shape or open aperture exposed to blood flow. For example, Figures 6A-6C schematically illustrate an anti-thrombus feature 500 disposed within a blind hole or aperture 544 of a connector 540. As described herein, the connector 540 may be used in place of or as a substitute for the connector 240 described above, and thus may be used interchangeably with the connector 240 and/or the insert 242 throughout the disclosure, for example. Similarly, the anti-thrombus feature 500 may be the same as or similar to the anti-thrombus feature 400 in construction, substance, materials, function, and the like.

For the purpose of illustration, the aperture 544 of Figures 6A-6C includes threads disposed therein. As discussed above with respect to the connector 240, other means of securing and/or attaching a delivery device to the connector 540 are also possible. In some embodiments, a delivery device may include an elongate rod 560 having a distal end configured to releasably attach to the connector 540. In some embodiments, the distal end of the elongate rod 560 may include threads configured to matingly align with the threads of the aperture 544. Similarly, in some embodiments, the distal end of the elongate rod 560 may include one or more features configured to engage other interlocking features disposed within the blind hole or aperture 544.

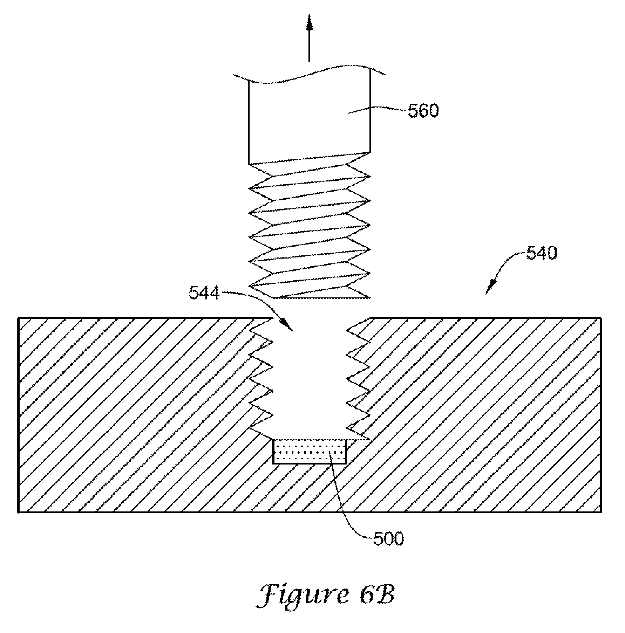

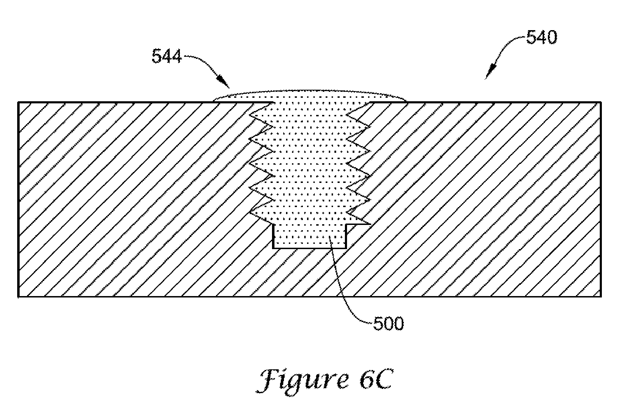

In some embodiments, the anti-thrombus feature 500 may be disposed within the aperture 544 of the connector 540, and the delivery device may be releasably attached to the connector 540, prior to percutaneous or minimally-invasive insertion, for example, to produce an installation condition of a medical device, such as the expandable filter device 200. In the installation condition, the anti-thrombus feature 500 may be disposed between the connector 540 and the distal end of the elongate rod 560 in an undeployed state, as shown in Figure 6A, such that the anti-thrombus feature 500 is protected from exposure to fluid (i.e., blood), which may cause the anti-thrombus feature 500 to swell and/or increase in volume. The skilled artisan will recognize that other arrangements of the anti-thrombus feature 500, for example, a coating disposed on the threads of the aperture 544, are also possible. Following percutaneous or minimally-invasive insertion of the medical device, the distal end of the elongate rod 560 may be withdrawn from the aperture 544, as seen in Figure 6B. Following withdrawal and/or removal of the distal end of the elongate rod 560 from the aperture 544, the anti-thrombus feature 500 may be exposed to blood flow. The anti-thrombus feature 500 may swell and/or increase in volume, in response to exposure to fluid (i.e., blood), to a deployed state, as may be seen in Figure 6C, to fill in the aperture 544 and/or to provide a smooth, protective surface over the aperture 544 which may reduce the potential for thrombus formation.

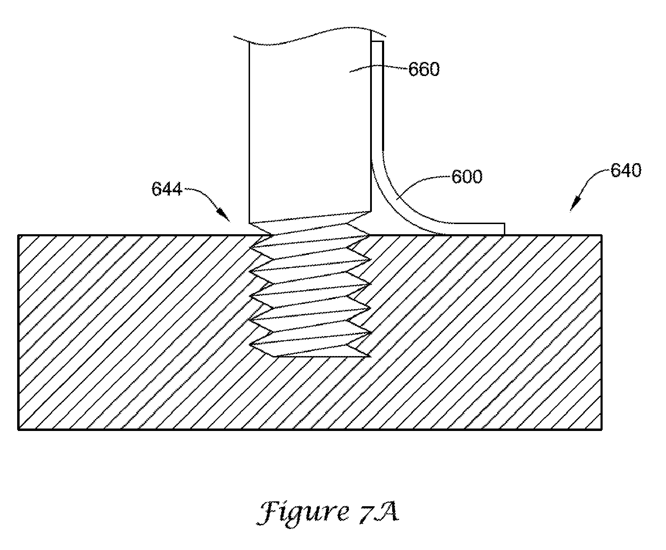

Figures 7A-7B schematically illustrate an anti-thrombus feature 600 disposed over or adjacent to a blind hole or aperture 644 of a connector 640. As described herein, the connector 640 may be used in place of or as a substitute for the connector 240 described above, and thus may be used interchangeably with the connector 240 and/or the insert 242 throughout the disclosure, for example.

For the purpose of illustration, the aperture 644 of Figures 7A-7B includes threads disposed therein. As discussed above with respect to the connector 240, other means of securing and/or attaching a delivery device to the connector 640 are also possible. In some embodiments, a delivery device may include an elongate rod 660 having a distal end configured to releasably attach to the connector 640. In some embodiments, the distal end of the elongate rod 660 may include threads configured to matingly align with the threads of the aperture 644. Similarly, in some embodiments, the distal end of the elongate rod 660 may include one or more features configured to engage other interlocking features disposed within the blind hole or aperture 644.

In some embodiments, the anti-thrombus feature 600 may include a self-biased flap attached to the connector 640 immediately adjacent the aperture 644. In some embodiments, the self-biased flap may include or be formed from a polymer, a metallic film or alloy(s) such as stainless steel (e.g. 304v stainless steel or 316L stainless steel), nickel-titanium alloy (e.g., nitinol, such as super elastic or linear elastic nitinol), nickel-chromium alloy, nickel-chromium-iron alloy, cobalt alloy, nickel, titanium, platinum, combinations thereof, or other suitable material(s) that provide sufficient spring force or bias to translate the anti-thrombus feature 600 from an undeployed state into a deployed state against, or in some embodiments with the assistance of, blood flow over the aperture 644 of the medical device.

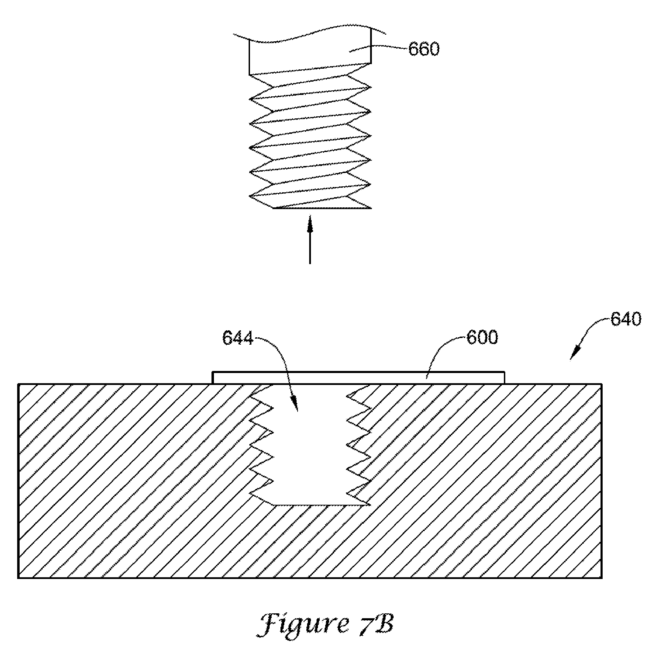

In some embodiments, the delivery device may be releasably attached to the connector 640, prior to percutaneous or minimally-invasive insertion, for example, to produce an installation condition of a medical device, such as the expandable filter device 200. In the installation condition, the anti-thrombus feature 600 may be held in the undeployed state by the elongate rod 660, as shown in Figure 7A. Following percutaneous or minimally-invasive insertion of the medical device, the elongate rod 660 may be withdrawn from the aperture 644, permitting the anti-thrombus feature 600 to translate into the deployed state, wherein the anti-thrombus feature 600 covers the aperture 644, as seen in Figure 7B. Following withdrawal and/or removal of the elongate rod 660 from the aperture 644, the anti-thrombus feature 600 may provide a smooth, protective surface over the aperture 644 which may reduce the potential for thrombus formation.

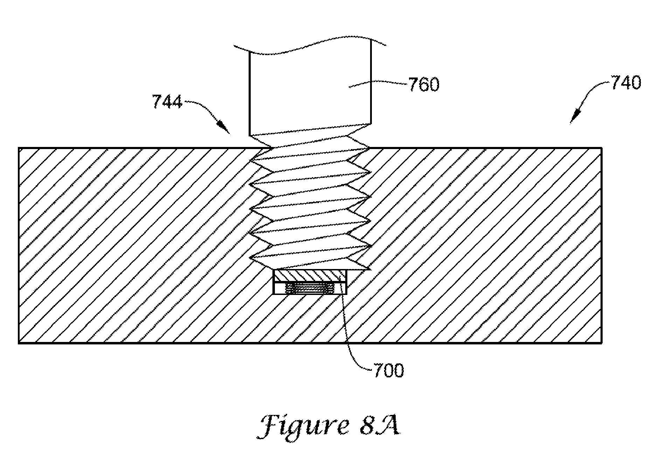

Figures 8A-8B schematically illustrate an anti-thrombus feature 700 disposed within a blind hole or aperture 744 of a connector 740. As described herein, the connector 740 may be used in place of or as a substitute for the connector 240 described above, and thus may be used interchangeably with the connector 240 and/or the insert 242 throughout the disclosure, for example.

For the purpose of illustration, the aperture 744 of Figures 8A-8B includes threads disposed therein. As discussed above with respect to the connector 240, other means of securing and/or attaching a delivery device to the connector 740 are also possible. In some embodiments, a delivery device may include an elongate rod 760 having a distal end configured to releasably attach to the connector 740. In some embodiments, the distal end of the elongate rod 760 may include threads configured to matingly align with the threads of the aperture 744. Similarly, in some embodiments, the distal end of the elongate rod 760 may include one or more features configured to engage other interlocking features disposed within the blind hole or aperture 744.

In some embodiments, the anti-thrombus feature 700 may include a spring-loaded cap disposed within and secured to the aperture 744 of the connector 740. In some embodiments, the anti-thrombus feature 700 may include or be formed from a polymer, a metallic film or alloy(s) such as stainless steel (e.g. 304v stainless steel or 316L stainless steel), nickel-titanium alloy (e.g., nitinol, such as super elastic or linear elastic nitinol), nickel-chromium alloy, nickel-chromium-iron alloy, cobalt alloy, nickel, titanium, platinum, combinations thereof, or other suitable material(s), and may include a spring such as a coil spring, a leaf spring, a bi-stable element, or other suitable means that provides sufficient spring force or bias to translate the anti-thrombus feature 700 from an undeployed state into a deployed state. In some embodiments, the anti-thrombus feature 700 may include or be formed from a swellable or expandable material such as a hydrogel or other materials similar to the anti-thrombus feature 400 described above.

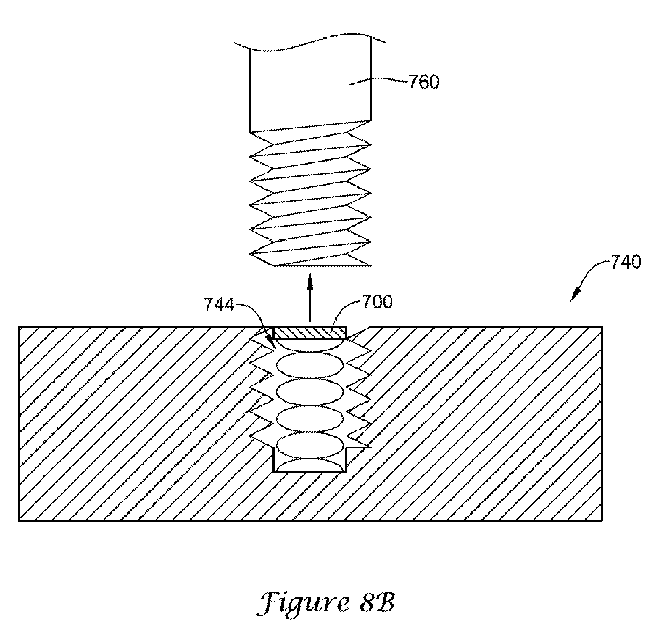

In some embodiments, the delivery device may be releasably attached to the connector 740, prior to percutaneous or minimally-invasive insertion, for example, to produce an installation condition of a medical device, such as the expandable filter device 200. In the installation condition, the anti-thrombus feature 700 may be held in the undeployed state by the distal end of the elongate rod 760, as shown in Figure 8A. Following percutaneous or minimally-invasive insertion of the medical device, the distal end of the elongate rod 760 may be withdrawn from the aperture 744, permitting the anti-thrombus feature 700 to translate into the deployed state, as seen in Figure 8B. Following withdrawal and/or removal of the elongate rod 760 from the aperture 744, the anti-thrombus feature 700 may provide a smooth, protective surface at a mouth of the aperture 744, effectively blocking off the aperture 744 from blood flow, which may reduce the potential for thrombus formation.

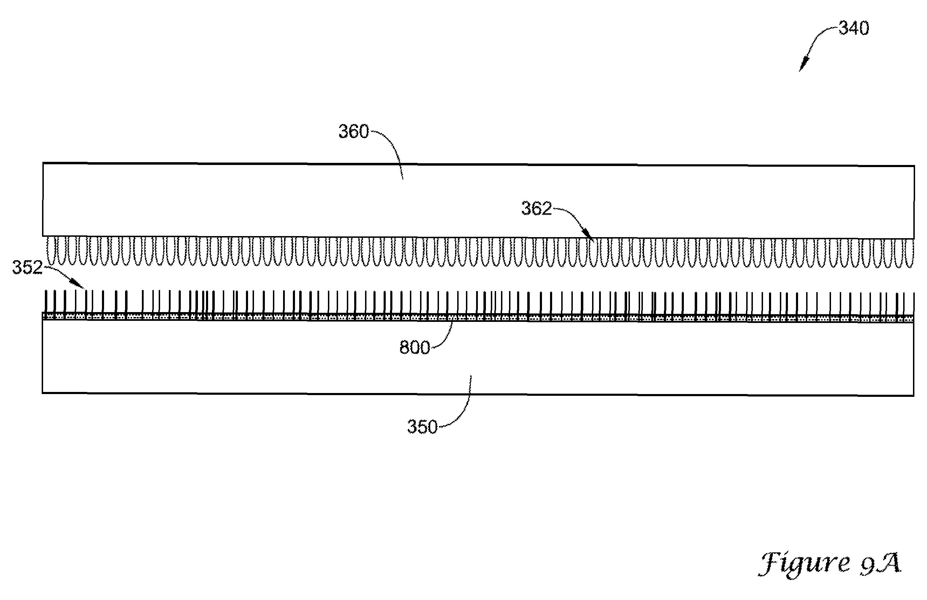

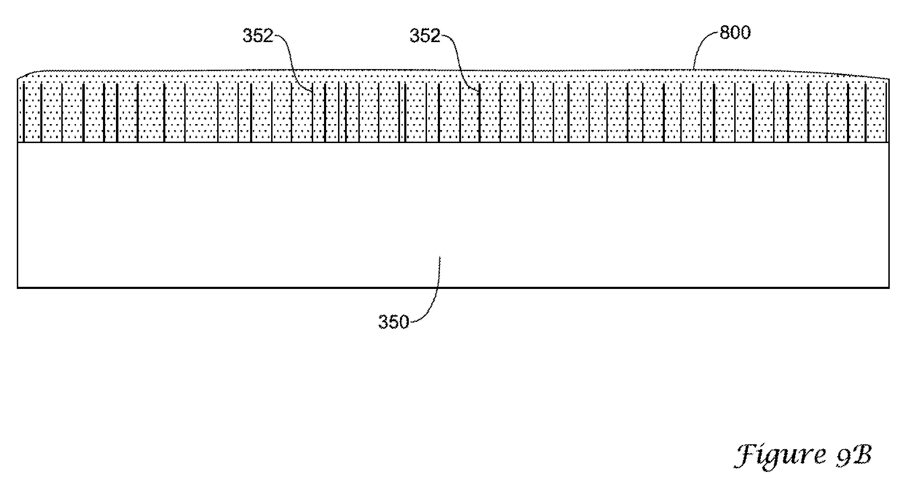

Figure 9A illustrates the example connector 340 of Figures 4 and 4A above including an anti-thrombus feature 800. As illustrated, the anti-thrombus feature 800 is shown as a thin coating disposed in, on, over, or around the plurality of hooks 352 of the first piece of material 350, in an undeployed state. In some embodiments, the anti-thrombus feature 800 may be disposed on surfaces of the connector 340 that are only exposed after the delivery device (not shown) and/or the second piece of material 360 has been removed. In some embodiments, the anti-thrombus feature 800 may include a swellable hydrogel and/or a water-swellable polymer that swells when it becomes hydrated (i.e., when the polymer is exposed to an aqueous environment such as body fluid(s), blood, and/or tissue). Some examples of swellable polymers may include polyvinyl alcohol, polyvinylpyrrolidone (PVP), polyethylene glycol, polyethylene oxide, hydroxypropyl methylcellulose, poly(hydroxyalkyl methacrylate) polyacrylic acid, coatings made of or including charged polymers, and the like. In some embodiments, the anti-thrombus feature 800 may swell and/or increase in volume upon exposure to fluid(s), such as blood or water, to a deployed state, as may be seen in Figure 9B. In some embodiments, the anti-thrombus feature 800 in the deployed state may increase in volume by 50%, 75%, 100%, 200%, 400%, 1000%, or more, upon exposure to fluid(s) compared to the undeployed state. In use, following insertion of the medical device 300 and actuation into the delivered condition, wherein the first piece of material 350 is separated from the second piece of material 360, as seen in Figure 9A, the anti-thrombus feature 800 may be exposed to fluid(s)/blood flow. In the delivered condition, the second piece of material 360 may be removed from the patient, leaving the plurality of hooks 352 of the first piece of material 350 exposed to blood flow as an irregular shape. The anti-thrombus feature 800 may then swell and/or increase in volume, in response to exposure to fluid (i.e., blood), to the deployed state, as may be seen in Figure 9B, to provide a smooth, protective surface over and/or around the plurality of hooks 352 and/or the irregular shape which may reduce the potential for thrombus formation. As may be appreciated, in some embodiments, the first piece of material 350 and the second piece of material 360 may be reversed, such that in the delivered condition, the first piece of material 350 may be removed from the patient, leaving the plurality of loops 362 of the second piece of material 360 exposed to blood flow as an irregular shape. The anti-thrombus feature 800 may then swell and/or increase in volume, in response to exposure to fluid (i.e., blood), to the deployed state, to provide a smooth, protective surface over and/or around the plurality of loops 362 and/or the irregular shape which may reduce the potential for thrombus formation.

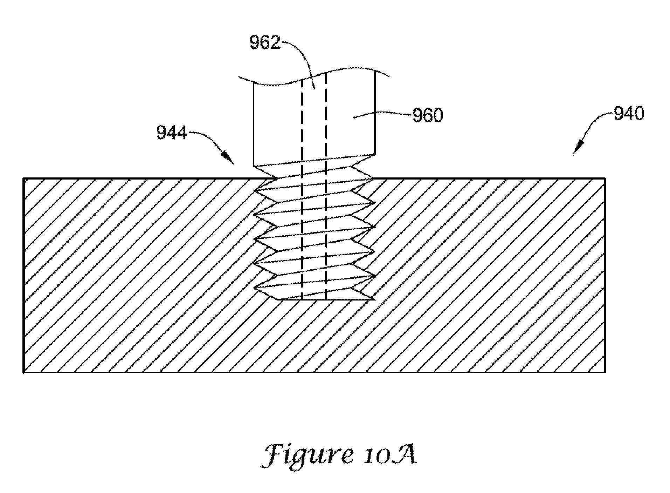

Figures 10A-10B schematically illustrate a connector 940 having a blind hole or aperture 944 disposed therein. In some embodiments, an anti-thrombus feature 900 may be inserted, applied, or added to the connector 940 in vivo, or while the connector 940 is disposed within a patient. As described herein, the connector 940 may be used in place of or as a substitute for the connector 240 described above, and thus may be used interchangeably with the connector 240 and/or the insert 242 throughout the disclosure, for example. Similarly, an anti-thrombus feature 900 may be the same as or similar to the anti-thrombus feature 400 in construction, substance, materials, function, and the like.

For the purpose of illustration, the aperture 944 of Figures 10A-10B includes threads disposed therein. As discussed above with respect to the connector 240, other means of securing and/or attaching a delivery device to the connector 940 are also possible. In some embodiments, a delivery device may include an elongate rod 960 having a distal end configured to releasably attach to the connector 940. In some embodiments, the distal end of the elongate rod 960 may include threads configured to matingly align with the threads of the aperture 944. Similarly, in some embodiments, the distal end of the elongate rod 960 may include one or more features configured to engage other interlocking features disposed within the blind hole or aperture 944. In some embodiments, the elongate rod 960 may include a lumen 962 extending therethrough, the lumen 962 being in fluid communication with the aperture 944 and a source (not shown) of the anti-thrombus feature 900.

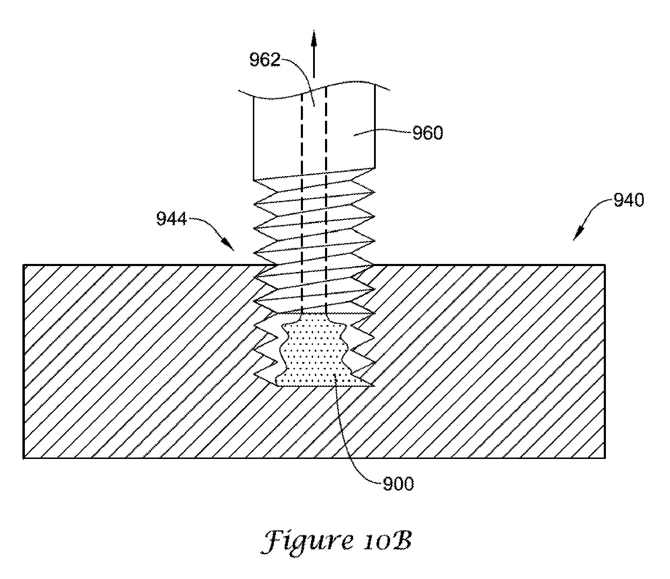

In some embodiments, the delivery device may be releasably attached to the connector 940, prior to percutaneous or minimally-invasive insertion, for example, to produce an installation condition of a medical device, such as the expandable filter device 200. In the installation condition, the anti-thrombus feature 900 may not be present within the connector 940, and thus in an undeployed state, as shown in Figure 10A, the anti-thrombus feature 900 is protected from exposure to fluid (i.e., blood) which may cause the anti-thrombus feature 900 to swell and/or increase in volume. Following percutaneous or minimally-invasive insertion of the medical device, the distal end of the elongate rod 960 may be withdrawn from the aperture 944, as seen in Figure 10B. During withdrawal and/or removal of the distal end of the elongate rod 960 from the aperture 944, the anti-thrombus feature 900 may be transferred from the source of the anti-thrombus feature 900, through the lumen 962, and into the aperture 944. Following withdrawal and/or removal of the elongate rod 960 from the aperture 944, the anti-thrombus feature 900 may be exposed to fluid/blood flow in a deployed state. In some embodiments, the anti-thrombus feature 900 may additionally swell and/or increase in volume, in response to exposure to fluid (i.e., blood), to define the deployed state. In the deployed state, the anti-thrombus feature 900 may fill in the aperture 944 and/or provide a smooth, protective surface over the aperture 944 which may reduce the potential for thrombus formation, similar to or the same as may be seen in Figure 6C.

In some embodiments, the anti-thrombus feature(s) described herein may be mixed with, loaded with, doped with, coated with, or otherwise include a pro-healing agent or drug (i.e., Annexin A5, etc.), an anti-thrombogenic substance (i.e., heparin, heparin derivatives, etc.), urokinase, D-phenylalanyl-L-prolyl-L-arginine chloromethyl ketone (PPACK), or other suitable treatment agents.

It should be understood that although the above discussion was focused on medical devices and methods of use within the vascular system of a patient, other embodiments of medical devices or methods in accordance with the disclosure can be adapted and configured for use in other parts of the anatomy of a patient. For example, devices and methods in accordance with the disclosure can be adapted for use in the digestive or gastrointestinal tract, such as in the mouth, throat, small and large intestine, colon, rectum, and the like. For another example, devices and methods can be adapted and configured for use within the respiratory tract, such as in the mouth, nose, throat, bronchial passages, nasal passages, lungs, and the like. Similarly, the apparatus and/or medical devices described herein with respect to percutaneous deployment may be used in other types of surgical procedures as appropriate. For example, in some embodiments, the medical devices may be deployed in a non-percutaneous procedure, such as an open-heart procedure. Devices and methods in accordance with the invention can also be adapted and configured for other uses within the anatomy.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A medical device for permanent implantation in a circulatory system of a patient, comprising:
an implant configured for percutaneous or minimally-invasive insertion into the patient;
at least one connector (140; 240; 340; 540; 640; 740; 940) fixedly attached to the implant and configured to releasably attach to a delivery device, the at least one connector (140; 240; 340; 540; 640; 740; 940) being exposed to blood flow within the circulatory system following implantation; and
an anti-thrombus feature (400; 500; 600; 700; 800; 900) configured to transition from an undeployed state to a deployed state.

2. The medical device of claim 1, wherein the at least one connector (140; 240; 340; 540; 640; 740; 940) includes an irregular shape exposed to blood flow; and wherein the anti-thrombus feature (400; 500; 600; 700; 800; 900) is disposed over the irregular shape in the undeployed state.

3. The medical device of claim 2, wherein the anti-thrombus feature (400; 500; 600; 700; 800; 900) increases in volume upon exposure to blood to the deployed state, the increase in volume is by at least 50%, preferably by at least 100%, more preferably by at least 500%.

4. The medical device of claim 2, wherein the anti-thrombus feature (400; 500; 600; 700; 800; 900) includes a coating disposed on surfaces of the at least one connector that are exposed to blood flow after the delivery device has been detached therefrom; and wherein the anti-thrombus feature (400; 500; 600; 700; 800; 900) forms a smooth surface over the irregular shape in the deployed state.

5. The medical device of claim 1, wherein the at least one connector (140; 240; 340; 540; 640; 740; 940) includes an open aperture (244; 544; 644; 744; 944) exposed to blood flow, wherein the anti-thrombus feature (400; 500; 600; 700; 800; 900) is disposed within the open aperture in the undeployed state.

6. The medical device of claim 1, wherein the delivery device includes an elongate rod (560; 660; 760; 960) having a distal end configured to releasably attach to the at least one connector (140; 240; 340; 540; 640; 740; 940) in an installation condition, the distal end of the elongate rod (560; 660; 760; 960) being configured to detach from the at least one connector (140; 240; 340; 540; 640; 740; 940) following implantation to produce a delivered condition.

7. The medical device of claim 5, wherein the anti-thrombus feature (400; 500; 600; 700; 800; 900) includes a material configured to increase in volume to the deployed state in response to exposure to blood; wherein the material fills the open aperture (244; 544; 644; 744; 944) and provides a smooth surface over the open aperture (244; 544; 644; 744; 944) in the deployed state, the material includes a hydrogel.

8. The medical device of claim 5, wherein the anti-thrombus feature (400; 500; 600; 700; 800; 900) includes a spring-loaded cap, the spring-loaded cap being configured to substantially block the open aperture (244; 544; 644; 744; 944) in the deployed state.

9. The medical device of claim 1, wherein the at least one connector (140; 240; 340; 540; 640; 740; 940) includes a first piece of material having a plurality of hooks (352) disposed thereon and a second piece of material having a plurality of loops (362) thereon;
wherein the plurality of hooks (352) is matingly engaged to the plurality of loops (362) in an installation condition;
wherein the anti-thrombus feature (400; 500; 600; 700; 800; 900) is disposed on the first piece of material in the undeployed state in the installation condition.

10. The medical device of claim 9, wherein in a delivered condition the plurality of loops (362) has been disengaged from the plurality of hooks (352) and the second piece of material has been separated from the first piece of material.

11. The medical device of claim 10, wherein in the delivered condition the anti-thrombus feature (400; 500; 600; 700; 800; 900) is configured to increase in volume to the deployed state in response to exposure to blood, such that the anti-thrombus feature (400; 500; 600; 700; 800; 900) covers the plurality of hooks (352) with a smooth surface in the deployed state.

12. The medical device of claim 6, wherein the elongate rod (560; 660; 760; 960) includes a lumen (962) extending therethrough;
wherein the anti-thrombus feature (400; 500; 600; 700; 800; 900) is initially disposed remote to the open aperture (244; 544; 644; 744; 944) in the undeployed state; and
wherein the lumen (962) is in fluid communication with a source of the anti-thrombus feature (400; 500; 600; 700; 800; 900).

13. The medical device of claim 12, wherein as the elongate rod (560; 660; 760; 960) is detached from the at least one connector (140; 240; 340; 540; 640; 740; 940), the anti-thrombus feature (400; 500; 600; 700; 800; 900) is transferred from the source through the lumen (962) and into the open aperture (244; 544; 644; 744; 944); and wherein the anti-thrombus feature (400; 500; 600; 700; 800; 900) includes a material configured to increase in volume to the deployed state in response to exposure to blood.

14. The medical device of claim 13, wherein the material fills the open aperture (244; 544; 644; 744; 944) and provides a smooth surface over the open aperture (244; 544; 644; 744; 944) in the deployed state.

15. The medical device of claim 6, wherein the anti-thrombus feature (400; 500; 600; 700; 800; 900) includes a flap self-biased toward covering the open aperture (244; 544; 644; 744; 944); wherein the flap is maintained in the undeployed state by the elongate rod (560; 660; 760; 960) in the installation condition; and wherein detachment of the elongate rod (560; 660; 760; 960) permits the flap to translate into the deployed state wherein the flap covers the open aperture (244; 544; 644; 744; 944).

## Patentansprüche

1. Medizinische Vorrichtung zur dauerhaften Implantation in ein Kreislaufsystem eines Patienten, die aufweist:
ein Implantat, das zur perkutanen oder minimal-invasiven Einführung in den Patienten konfiguriert ist;
mindestens ein Verbindungsstück (140; 240; 340; 540; 640; 740; 940), das fest am Implantat angebracht ist und konfiguriert ist, lösbar an einer Zuführungsvorrichtung angebracht zu werden, wobei das mindestens eine Verbindungsstück (140; 240; 340; 540; 640; 740; 940) anschließend an die Implantation dem Blutfluss innerhalb des Kreislauf systems ausgesetzt ist; und
ein antithrombotisches Merkmal (400; 500; 600; 700; 800; 900), das konfiguriert ist, von einem nicht entfalteten Zustand in einen entfalteten Zustand überzugehen.

2. Medizinische Vorrichtung nach Anspruch 1, wobei das mindestens eine Verbindungsstück (140; 240; 340; 540; 640; 740; 940) eine unregelmäßige Form aufweist, die dem Blutfluss ausgesetzt ist; und wobei das antithrombotische Merkmal (400; 500; 600; 700; 800; 900) im nicht entfalteten Zustand über der unregelmäßigen Form angeordnet ist.

3. Medizinische Vorrichtung nach Anspruch 2, wobei das antithrombotische Merkmal (400; 500; 600; 700; 800; 900), wenn es Blut ausgesetzt wird, sein Volumen auf den entfalteten Zustand erhöht, wobei die Zunahme des Volumens mindestens 50%, vorzugsweise mindestens 100%, besonders bevorzugt mindestens 500% beträgt.

4. Medizinische Vorrichtung nach Anspruch 2, wobei das antithrombotische Merkmal (400; 500; 600; 700; 800; 900) eine Beschichtung aufweist, die auf Oberflächen des mindestens einen Verbindungsstücks angeordnet ist, die dem Blutfluss ausgesetzt sind, nachdem die Zuführungsvorrichtung davon gelöst worden ist; und wobei das antithrombotische Merkmal (400; 500; 600; 700; 800; 900) im entfalteten Zustand eine glatte Oberfläche über der unregelmäßigen Form bildet.

5. Medizinische Vorrichtung nach Anspruch 1, wobei das mindestens eine Verbindungsstück (140; 240; 340; 540; 640; 740; 940) eine durchlässige Öffnung (244; 544; 644; 744; 944) aufweist, die dem Blutfluss ausgesetzt ist, wobei das antithrombotische Merkmal (400; 500; 600; 700; 800; 900) im nicht entfalteten Zustand in der durchlässigen Öffnung angeordnet ist.

6. Medizinische Vorrichtung nach Anspruch 1, wobei die Zuführungsvorrichtung einen länglichen Stab (560; 660; 760; 960) aufweist, der ein distales Ende aufweist, das konfiguriert ist, in einem Installationszustand lösbar an dem mindestens einen Verbindungsstück (140; 240; 340; 540; 640; 740; 940) angebracht zu sein, wobei das distale Ende des länglichen Stabs (560; 660; 760; 960) konfiguriert ist, sich anschließend an die Implantation von dem mindestens einen Verbindungsstück (140; 240; 340; 540; 640; 740; 940) zu lösen, um einen Abgabezustand zu erzeugen.

7. Medizinische Vorrichtung nach Anspruch 5, wobei das antithrombotische Merkmal (400; 500; 600; 700; 800; 900) ein Material aufweist, das konfiguriert ist, sein Volumen als Reaktion darauf, Blut ausgesetzt zu sein, zum entfalteten Zustand zu erhöhen; wobei das Material die durchlässige Öffnung (244; 544; 644; 744; 944) füllt und im entfalteten Zustand eine glatte Oberfläche über der durchlässigen Öffnung (244; 544; 644; 744; 944) bereitstellt, wobei das Material ein Hydrogel aufweist.

8. Medizinische Vorrichtung nach Anspruch 5, wobei das antithrombotische Merkmal (400; 500; 600; 700; 800; 900) eine federbelastete Kappe aufweist, wobei die federbelastete Kappe konfiguriert ist, die durchlässige Öffnung (244; 544; 644; 744; 944) im entfalteten Zustand im Wesentlichen zu blockieren.

9. Medizinische Vorrichtung nach Anspruch 1, wobei das mindestens eine Verbindungsstück (140; 240; 340; 540; 640; 740; 940) ein erstes Materialstück, das mehrere daran angeordnete Haken (352) aufweist, und ein zweites Materialstück aufweist, das mehrere Schlingen (362) daran aufweist;
wobei die mehreren Haken (352) in einem Installationszustand mit den mehreren Schlingen (362) passend in Eingriff stehen;
wobei das antithrombotische Merkmal (400; 500; 600; 700; 800; 900) im nicht entfalteten Zustand im Installationszustand am ersten Materialstück angeordnet ist.

10. Medizinische Vorrichtung nach Anspruch 9, wobei in einem Abgabezustand die mehreren Schlingen (362) von den mehreren Haken (352) gelöst worden sind und das zweite Materialstück vom ersten Materialstück getrennt worden ist.

11. Medizinische Vorrichtung nach Anspruch 10, wobei im Abgabezustand das antithrombotische Merkmal (400; 500; 600; 700; 800; 900) konfiguriert ist, als Reaktion darauf, Blut ausgesetzt zu sein, sein Volumen zum entfalteten Zustand so zu erhöhen, dass das antithrombotische Merkmal (400; 500; 600; 700; 800; 900) im entfalteten Zustand die mehreren der Haken (352) mit einer glatten Oberfläche bedeckt.

12. Medizinische Vorrichtung nach Anspruch 6, wobei der längliche Stab (560; 660; 760; 960) ein sich dort hindurch erstreckendes Lumen (962) aufweist;
wobei das antithrombotische Merkmal (400; 500; 600; 700; 800; 900) im nicht entfalteten Zustand anfänglich entfernt von der durchlässigen Öffnung (244; 544; 644; 744; 944) angeordnet ist; und
wobei das Lumen (962) in Fluidverbindung mit einer Quelle des antithrombotischen Merkmals (400; 500; 600; 700; 800; 900) steht.

13. Medizinische Vorrichtung nach Anspruch 12, wobei, wenn der längliche Stab (560; 660; 760; 960) von dem mindestens einen Verbindungsstück (140; 240; 340; 540; 640; 740; 940) gelöst wird, das antithrombotische Merkmal (400; 500; 600; 700; 800; 900) von der Quelle durch das Lumen (962) und in die durchlässige Öffnung (244; 544; 644; 744; 944) überführt wird; und wobei das antithrombotische Merkmal (400; 500; 600; 700; 800; 900) ein Material aufweist, das konfiguriert ist, als Reaktion darauf, Blut ausgesetzt zu sein, sein Volumen zum entfalteten Zustand zu erhöhen.

14. Medizinische Vorrichtung nach Anspruch 13, wobei das Material die durchlässige Öffnung (244; 544; 644; 744; 944) ausfüllt und im entfalteten Zustand eine glatte Oberfläche über der durchlässigen Öffnung (244; 544; 644; 744; 944) bereitstellt.

15. Medizinische Vorrichtung nach Anspruch 6, wobei das antithrombotische Merkmal (400; 500; 600; 700; 800; 900) eine Klappe aufweist, die zur Abdeckung der durchlässigen Öffnung (244; 544; 644; 744; 944) selbstvorgespannt ist; wobei die Klappe im Installationszustand durch den länglichen Stab (560; 660; 760; 960) im nicht entfalteten Zustand gehalten wird; und wobei das Lösen des länglichen Stabs (560; 660; 760; 960) es der Klappe ermöglicht, sich in den entfalteten Zustand zu verschieben, wobei die Klappe die durchlässige Öffnung (244; 544; 644; 744; 944) bedeckt.

## Revendications

1. Dispositif médical destiné à une implantation permanente dans le système circulatoire d'un patient, comprenant :
un implant prévu pour une insertion percutanée ou mini-invasive dans le corps du patient ;
au moins un connecteur (140 ; 240 ; 340 ; 540 ; 640 ; 740 ; 940) raccordé de manière fixe à l'implant et prévu pour une fixation amovible à un dispositif d'administration, ledit au moins un connecteur (140 ; 240 ; 340 ; 540 ; 640 ; 740 ; 940) étant exposé au flux sanguin dans le système circulatoire après implantation ; et
un élément anti-thrombique (400 ; 500 ; 600 ; 700 ; 800 ; 900) prévu pour passer d'un état non déployé à un état déployé.

2. Dispositif médical selon la revendication 1, où ledit au moins un connecteur (140 ; 240 ; 340 ; 540 ; 640 ; 740 ; 940) présente une forme irrégulière exposée au flux sanguin ; et où l'élément anti-thrombique (400 ; 500 ; 600 ; 700 ; 800 ; 900) est disposé sur la forme irrégulière en état non déployé.

3. Dispositif médical selon la revendication 2, où le volume de l'élément anti-thrombique (400 ; 500 ; 600 ; 700 ; 800 ; 900) augmente par exposition au sang pour passer à l'état déployé, l'augmentation de volume étant d'au moins 50 %, avantageusement d'au moins 100 %, préférentiellement d'au moins 500 %.

4. Dispositif médical selon la revendication 2, où l'élément anti-thrombique (400 ; 500 ; 600 ; 700 ; 800 ; 900) présente un revêtement sur des surfaces dudit au moins un connecteur exposées au flux sanguin après détachement du dispositif d'administration ; et où l'élément anti-thrombique (400 ; 500 ; 600 ; 700 ; 800 ; 900) forme une surface lisse sur la forme irrégulière en état déployé.

5. Dispositif médical selon la revendication 1, où ledit au moins un connecteur (140 ; 240 ; 340 ; 540 ; 640 ; 740 ; 940) présente une ouverture (244 ; 544 ; 644 ; 744 ; 944) exposée au flux sanguin, l'élément anti-thrombique (400 ; 500 ; 600 ; 700 ; 800 ; 900) étant disposé dans l'ouverture en état non déployé.

6. Dispositif médical selon la revendication 1, où le dispositif d'administration comporte une tige allongée (560 ; 660 ; 760 ; 960) ayant une extrémité distale prévue pour être fixée de manière amovible audit au moins un connecteur (140 ; 240 ; 340 ; 540 ; 640 ; 740 ; 940) en état d'installation, l'extrémité distale de la tige allongée (560 ; 660 ; 760 ; 960) étant prévue pour être détachée dudit au moins un connecteur (140 ; 240 ; 340 ; 540 ; 640 ; 740 ; 940) après implantation afin d'obtenir un état d'administration.

7. Dispositif médical selon la revendication 5, où l'élément anti-thrombique (400 ; 500 ; 600 ; 700 ; 800 ; 900) comprend un matériau prévu pour présenter une augmentation de volume vers l'état déployé en réaction à une exposition au sang ; où le matériau comble l'ouverture (244 ; 544 ; 644 ; 744 ; 944) et réalise une surface lisse sur l'ouverture (244 ; 544 ; 644 ; 744 ; 944) en état déployé, ledit matériau comprenant un hydrogel.

8. Dispositif médical selon la revendication 5, où l'élément anti-thrombique (400 ; 500 ; 600 ; 700 ; 800 ; 900) comporte un bouchon contraint par ressort, ledit bouchon contraint par ressort étant prévu pour obturer sensiblement l'ouverture (244 ; 544 ; 644 ; 744 ; 944) en état déployé.

9. Dispositif médical selon la revendication 1, où ledit au moins un connecteur (140 ; 240 ; 340 ; 540 ; 640 ; 740 ; 940) comprend une première pièce de matériau pourvue d'une pluralité de crochets (352) disposés sur elle et une deuxième pièce de matériau pourvue d'une pluralité de boucles (362) disposées sur elle ;
la pluralité de crochets (352) étant en prise complémentaire sur la pluralité de boucles (362) en état d'installation ;
l'élément anti-thrombique (400 ; 500 ; 600 ; 700 ; 800 ; 900) étant disposé sur la première pièce de matériau en état non déployé dans l'état d'installation.

10. Dispositif médical selon la revendication 9, où en état d'administration la pluralité de crochets (352) sont dégagés de la pluralité de boucles (362) et la deuxième pièce de matériau est séparée de la première pièce de matériau.

11. Dispositif médical selon la revendication 10, où en état d'administration l'élément anti-thrombique (400 ; 500 ; 600 ; 700 ; 800 ; 900) est prévu pour présenter une augmentation de volume vers l'état déployé en réaction à une exposition au sang, de sorte que l'élément anti-thrombique (400 ; 500 ; 600 ; 700 ; 800 ; 900) recouvre la pluralité de crochets (352) par une surface lisse en état déployé.

12. Dispositif médical selon la revendication 6, où la tige allongée (560 ; 660 ; 760 ; 960) présente une lumière (962) s'étendant en elle ;
où l'élément anti-thrombique (400 ; 500 ; 600 ; 700 ; 800 ; 900) est initialement disposé à distance de l'ouverture (244 ; 544 ; 644 ; 744 ; 944) en état non déployé ; et
où la lumière (962) est en communication fluidique avec une source de l'élément anti-thrombique (400 ; 500 ; 600 ; 700 ; 800 ; 900).

13. Dispositif médical selon la revendication 12, où quand la tige allongée (560 ; 660 ; 760 ; 960) est détachée dudit au moins un connecteur (140 ; 240 ; 340 ; 540 ; 640 ; 740 ; 940), l'élément anti-thrombique (400 ; 500 ; 600 ; 700 ; 800 ; 900) est transféré de la source à l'ouverture (244 ; 544 ; 644 ; 744 ; 944) par la lumière (962); et où l'élément anti-thrombique (400 ; 500 ; 600 ; 700 ; 800 ; 900) comprend un matériau prévu pour présenter une augmentation de volume vers l'état déployé en réaction à une exposition au sang.

14. Dispositif médical selon la revendication 13, où le matériau comble l'ouverture (244 ; 544 ; 644 ; 744 ; 944) et réalise une surface lisse sur l'ouverture (244 ; 544 ; 644 ; 744 ; 944) en état déployé.

15. Dispositif médical selon la revendication 6, où l'élément anti-thrombique (400 ; 500 ; 600 ; 700 ; 800 ; 900) est pourvu d'un rabat auto-contraint dans une direction de couverture de l'ouverture (244 ; 544 ; 644 ; 744 ; 944) ; où le rabat est maintenu en état non déployé par la tige allongée (560 ; 660 ; 760 ; 960) dans l'état d'installation ; et où la détachement de la tige allongée (560 ; 660 ; 760 ; 960) permet au rabat de passer en état déployé, le rabat recouvrant alors l'ouverture (244 ; 544 ; 644 ; 744 ; 944).
